# EUROPÄISCHE PATENTSCHRIFT

(11) **EP 0 676 390 B1**
(45) Veröffentlichungstag und Bekanntmachung des Hinweises auf die Patenterteilung: **02.01.1997**
(21) Anmeldenummer: 95101384.6
(22) Anmeldetag: 02.02.1995
(51) Int. Cl.: C07C 227/08, C07C 227/18, C07C 229/46, C07C 229/48

(54) **Verfahren zur Herstellung von 1-Aminocyclopropancarbonsäurehydrochlorid**
Process for the preparation of 1-aminocyclopropane carboxylic acid hydrochloride
Procédé pour la préparation d'hydrochlorure de l'acide 1-aminocyclopropane carboxylique

(30) Priorität: 06.04.1994 DE 4411777
(43) Veröffentlichungstag der Anmeldung: 11.10.1995
(73) Patentinhaber: HÜLS AKTIENGESELLSCHAFT, 45764 Marl (DE)
(72) Erfinder: Kleemiss, Wolfgang, Dr., D-45721 Haltern (DE); Feld, Marcel, Dr., D-51147 Köln (DE)

(56) Entgegenhaltungen:
- CHEMICAL ABSTRACTS, vol. 109, no. 15, 10.Oktober 1988 Columbus, Ohio, US; abstract no. 128440e, Seite 635; Spalte 1;
- JOURNAL OF THE CHEMICAL SOCIETY, Nr. 428, 1960 LETCHWORTH GB, Seiten 2119-2132, T.A. CONNORS ET AL. 'Some derivatives of 1-aminocyclopentanecarboxylic acid and related compounds'

## Beschreibung

Die Erfindung betrifft ein neues Verfahren zur Herstellung von 1-Aminocyclopropancarbonsäurehydrochlorid. 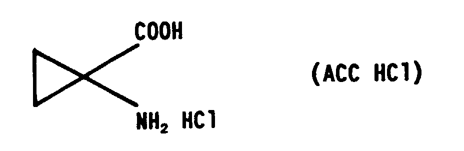 aus einem Cyclopropan-1,1-dicarbonsäurediester der Formel **1**

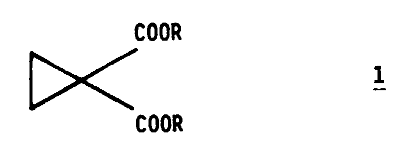

Für die Aminosäure 1-Aminocyclopropancarbonsäure (ACC), die leicht mittels Ionenaustauscher aus ACC HCl gewonnen werden kann, gibt es zahlreiche Verwendungsmöglichkeiten. So dient ACC beispielsweise als Wachstumsregulator für Pflanzen, indem es das Pflanzenwachstumshormon Ethylen enzynkatalysiert freisetzt. Außerdem wird ACC als Synthesebaustein für die Herstellung von Bakteriziden, Fungiziden und Insektiziden eingesetzt. Man sucht daher nach Verfahren, mit denen man ACC auf einfache Weise in guten Ausbeuten und in hoher Reinheit herstellen kann.

Verbindungen der Formel **1** sind leicht aus Malonsäurediestern und 1,2-Dibromethan bzw. 1,2-Dichlorethan herstellbar.

In SU 1 313 851 ist die Synthese von ACC, ausgehend vom Diester **1**, nach folgendem Schema beschrieben:

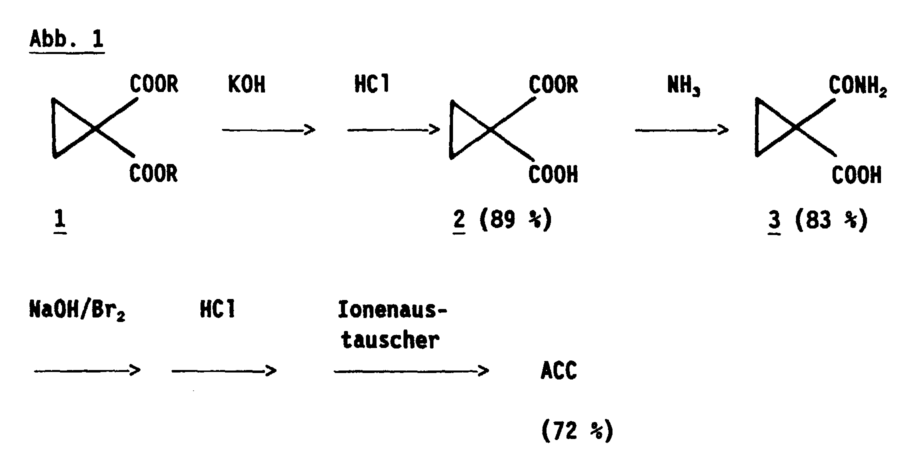

Das Verfahren ist jedoch sehr aufwendig und die Gesamtausbeute ist gering (ca. 50 %). In der ersten Stufe verseift man zum 1,1-Cyclopropancarbonsäuremonoester **2**, welcher anschließend mit Ammoniak zum Monoamid **3** umgesetzt wird. Dann wird der Hofmann-Abbau zum Natriumsalz der ACC durchgeführt. Die Aminosäure wird mittels Ionenaustauscher gewonnen.

Dieses Verfahren hat zahlreiche Nachteile. Erstens ist die Verseifung des Diesters **1** zum Monoester **2** sehr aufwendig. Man rührt den Diester für 2 Tage mit Kaliumhydroxid bei Raumtemperatur und stellt dann mit konzentrierter Salzsäure auf pH 1 ein. Der Monoester **2** wird dann mit Ethylacetat extrahiert. Zweitens muß vor der Umsetzung mit Ammoniak zum Monoamid **3** das Ethylacetat durch Destillation entfernt werden. Die Ausbeuten beider Reaktionsschritte betragen auch nur jeweils 89 % bzw. 83 %. Ein weiterer Nachteil des Verfahrens ist, daß der nachfolgende Hofmann-Abbau diskontinuierlich durchgeführt wird. Diese Fahrweise ist bei größeren Ansätzen im technischen Maßstab nicht realisierbar, da die Exothermie des Hofmann-Abbaus nicht kontrolliert werden kann. Wir verweisen dazu auf DE-A-38 36 917.

Eine weitere Synthese führt nach T. A. Connors et al., J. Chem. Soc. 2129 (1960) über 4 Stufen zur ACC:

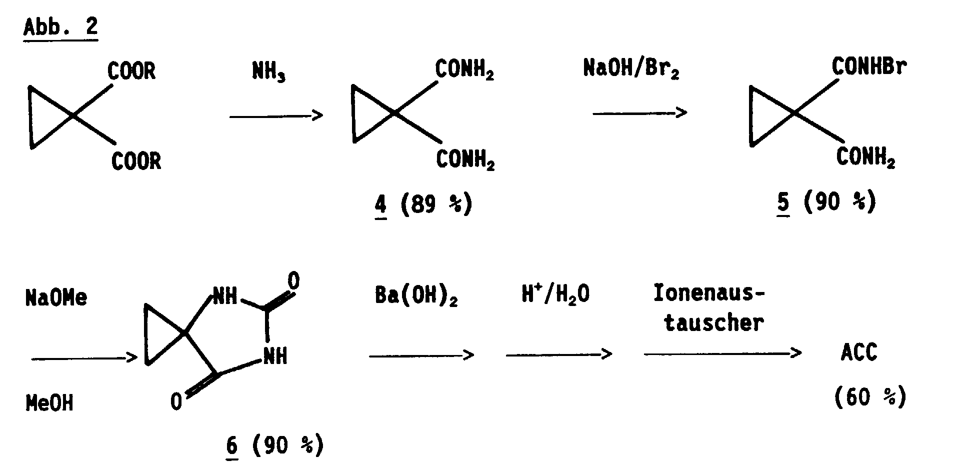

Diese 4stufige Synthese ist aufwendig und führt über schwer handhabbare Zwischenprodukte. Das Bisamid **4** ist schwerlöslich in Wasser, während das bromhaltige Produkt **5** eine sehr instabile Verbindung darstellt. Die Gesamtausbeute dieser Synthese beträgt nur 35 %.

Es bestand daher die Aufgabe, eine einfache Synthese von ACC HCl, ausgehend von Cyclopropan-1,1-dicarbonsäurediestern, auszuarbeiten, die in wenigen Stufen und guten Ausbeuten zum Produkt führt. Außerdem sollten die Zwischenprodukte stabile und gut handhabbare Verbindungen sein. Die kritische Stufe, der Hofmann-Abbau, sollte darüber hinaus kontinuierlich oder halbkontinuierlich durchgeführt werden können, um die Exothermie der Reaktion zu beherrschen.

Die Aufgabe wird erfindungsgemäß dadurch gelöst, daß man den Cyclopropan-1,1-dicarbonsäurediester der Formel **1** über die Zwischenprodukte 1-Aminocarbonylcyclopropancarbonsäureester der Formel 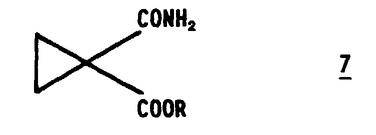 und 1-Aminocarbonylcyclopropancarbonsäuresalz der Formel 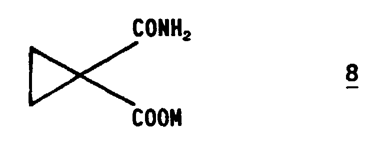 zu ACC HCl umsetzt. Dabei bedeuten R Alkyl mit 1 bis 8 C-Atomen und M Alkali- oder Erdalkalimetall. Außerdem wird die Reaktion vom Diester der Formel **1** zum Monoamidester der Formel **7** in einer Lösung eines Alkohols mit 1 bis 8 C-Atomen mit Ammoniak bei einer Temperatur von 0 bis 100 °C und einem NH₃-Druck von 1 bis 5 bar durchgeführt.

Anschließend erfolgt ein Hofmann-Abbau des Salzes **8** zum Alkali- bzw. Erdalkalisalz von ACC, welches über das Hydrochlorid gereinigt und gegebenenfalls in die freie Aminosäure übergeführt werden kann, so daß das Gesamtreaktionsschema gemäß Abb. 3 formuliert werden kann:

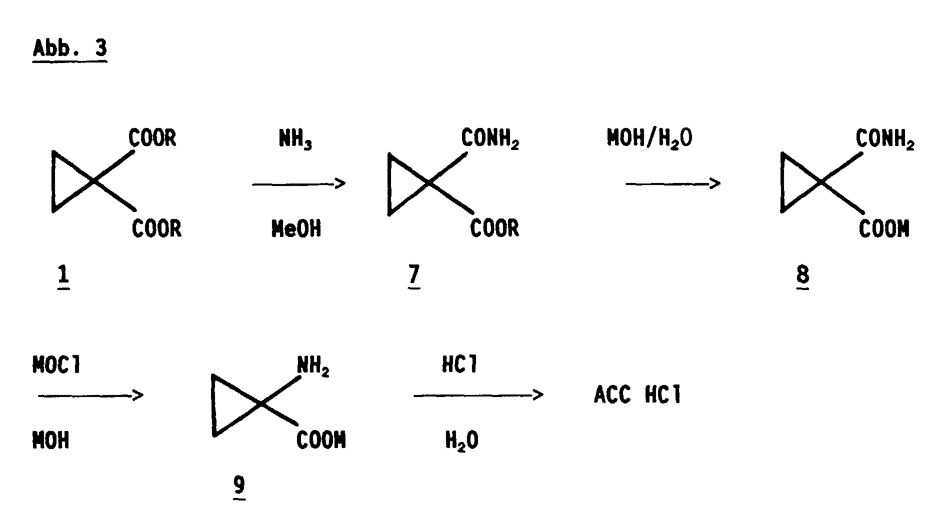

Überraschenderweise wurde gefunden, daß die Ausbeute der ersten Stufe > 90 % beträgt und die Verseifung zum Salz **8** praktisch quantitativ verläuft.

Ein besonderer Vorteil des Verfahrens ist, daß die Zwischenstufen **7**, **8** und **9** nicht isoliert werden müssen, sondern jeweils ohne Aufarbeitung direkt in die jeweils folgende Stufe eingehen können.

Geeignete Alkohole, die vorzugsweise den Esteralkoholen des Diesters **1** entsprechen, sind beispielsweise Methanol, Ethanol, Isopropanol, Butanol oder Hexanol.

In einer besonderen Ausführungsform der Erfindung wird die Amidierung des Diesters **1** durch Alkali- oder Erdalkalimetallalkoholate katalysiert. Beispiele für Alkalimetalle sind vor allem Lithium, Natrium und Kalium, für Erdalkalimetalle unter anderem Calcium und Barium.

Der Monoamidester 7 entsteht in einer Ausbeute von > 95 %. Interessanterweise gelingt die Amidierung des Diesters **1** auch ohne Alkoholat als Katalysator.

Zur alkoholischen Suspension des Monoamidesters **7** gibt man anschließend vorzugsweise ein Äquivalent einer wäßrigen Alkalihydroxidlösung. Das Gemisch wird auf eine Temperatur von 10 bis 100 °C erwärmt, wobei der Feststoff in Lösung geht. Gleichzeitig wird der Alkohol aus dem Gemisch herausdestilliert. Die Ausbeute der Verseifung ist praktisch quantitativ. Man erhält eine homogene wäßrige Lösung des Salzes **8**. Wird eine hochkonzentrierte Lösung eines Alkalihydroxids verwendet, kann nach der Hydrolyse mit Wasser verdünnt werden, um eine homogene Lösung zu erhalten.

Die wäßrige Lösung des Salzes **8** wird im allgemeinen direkt in den Hofmann-Abbau eingesetzt. Die Lösung wird dabei vorzugsweise kontinuierlich oder halbkontinuierlich mit einem Gemisch aus Alkalihypochlorit und Alkalihydroxidlösung bei 40 bis 150 °C bei Normaldruck oder bei einem Druck bis 5 bar umgesetzt. Dabei werden, bezogen auf das Salz **8**, meist 1 bis 1,5 Äquivalente Alkalihypochlorit und 1,1 bis 4 Äquivalente Alkalihydroxid eingesetzt. Geeignete Alkalimetalle sind dabei vor allem Lithium, Natrium und Kalium.

Der alkalische Austrag des Hofmann-Abbaus kann mit einer Salzsäurelösung auf einen pH 1 bis 2 eingestellt werden. Anschließend kann das Reaktionsgemisch eingedampft und das ACC HCl mit einem Alkohol, vorzugsweise Ethanol, aus dem Rückstand herausgelöst werden, um auf diese Weise vom Natriumchlorid abzutrennen. Die alkoholische Lösung von ACC HCl wird wiederum eingedampft. Letzte Verunreinigungen können z. B. mit heißem Aceton herausgewaschen werden. Man gelangt zu ACC HCl mit einer Ausbeute von ca. 80 %. Die freie Aminosäure ACC läßt sich nach dem Stand der Technik z. B. mit Hilfe von Ionenaustauschern aus dem ACC HCl gewinnen.

Bei der praktischen Durchführung des erfindungsgemäßen Verfahrens wird die Amidierung des Diesters **1** zum Monoamidester **7** vorzugsweise so durchgeführt, daß man den Diester **1** bei einer Temperatur von 20 bis 60 °C und einem NH₃-Druck von 1 bar umsetzt.

Als Lösungsmittel dient ein Alkohol, vorzugsweise der Esteralkohol des Diesters **1**.

Man kann die Amidierung dadurch beschleunigen, daß man 1 bis 40 mol-% eines Alkalialkoholats als Katalysator verwendet. Auch Erdalkalialkoholate, wie z. B. Magnesiumalkoholat, können als Katalysatoren eingesetzt werden. Der Alkohol des Alkoholats besitzt 1 bis 8 C-Atome, wobei bevorzugt Alkoholate niederer Alkohole verwendet werden.

In dem erfindungsgemäßen Verfahren wird weiterhin der Monoamidester **7** mit einer 1- bis 40%igen wäßrigen Lösung eines Alkalihydroxids bei einer Temperatur von vorzugsweise 25 bis 50 °C zum Salz **8** verseift, ohne dabei die Amidfunktion zu verseifen. Man kann zur alkoholischen Suspension des Monoamidesters **7** direkt die verdünnte Natronlauge geben und anschließend den Alkohol aus dem Gemisch herausdestillieren. Dabei erhält man eine wäßrige Lösung des Salzes **8**.

Ein Vorteil des erfindungsgemäßen Verfahrens ist, daß der Hofmann-Abbau des Salzes **8** kontinuierlich durchgeführt werden kann. Die wäßrige Lösung des Salzes **8** wird mit einem Gemisch aus einer 5- bis 14%igen Alkalihypochloritlösung und bevorzugt 1,5 bis 3 Äquivalenten einer 10- bis 50%igen Alkalihydroxidlösung bei einer Temperatur von 10 bis 60 °C zusammengeführt und anschließend z. B. durch ein Reaktionsrohr auf eine Temperatur von bevorzugt 60 bis 100 °C gebracht.

Der alkalische Reaktionsaustrag kann mit Salzsäure auf pH 1 bis 2 eingestellt werden. Durch Abdampfen des Wassers erhält man ein gegebenenfalls wäßriges Gemisch aus dem Hydrochlorid der Aminosäure und dem Alkalimetallchlorid.

Durch Extraktion mit Alkohol gewinnt man eine alkoholische Lösung von ACC HCl, die wiederum eingeengt wird. Letzte Verunreinigungen können z. B. mit Aceton aus dem Produkt gewaschen werden. ACC HCl wird in einer Ausbeute von meist > 75 % mit einer Reinheit von > 99 % erhalten.

### 1) Herstellung von 1-Aminocarbonylcyclopropancarbonsäuremethylester

In eine Lösung von 1 106 g (7 mol) Cyclopropandicarbonsäuredimethylester in 700 ml Methanol wird bei 20 °C unter Rühren Ammoniak (20 l/h) eingeleitet. Nach ca. 10 h beendet man die NH₃-Einleitung und kühlt das Reaktionsgemisch auf 8 °C. Der Feststoff wird abgesaugt und mit 200 g kaltem Methanol gewaschen. Nach der Trocknung des Produkts erhält man 926,2 g (92,5 %) 1-Aminocarbonylcyclopropancarbonsäuremethylester (Schmp.: 157 °C).

### 2) Herstellung von 1-Aminocarbonylcyclopropancarbonsäure

In eine Lösung von 1 106 g (7 mol) Cyclopropan-1,1-dicarbonsäuredimethylester in 700 ml Methanol wird bei 20 °C unter Rühren Ammoniak (20 l/h) eingeleitet. Nach ca. 10 h beendet man die NH₃-Einleitung.

Zu der Suspension gibt man 1,4 1 20%ige Natronlauge und erwärmt das Gemisch auf 40 °C. Im Vakuum wird das Methanol abdestilliert. Nach dem Erkalten fügt man 680 g konzentrierte Salzsäure hinzu, woraufhin das Produkt ausfällt. Es wird abgesaugt, mit kaltem Wasser gewaschen und getrocknet (Schmp.: 182 °C), Ausbeute: 813 g, 90 %.

Wenn die 1-Aminocarbonylcyclopropancarbonsäure zu ACC abgebaut werden soll, entfällt die Neutralisation mit Salzsäure. Die wäßrige Lösung des 1-Aminocarbonylcyclopropancarbonsäurenatriumsalzes wird dann direkt mit Natriumhypochlorit und Natronlauge umgesetzt [siehe: 3), Beispiel 2, Teil c].

### 3) Herstellung von 1-Aminocyclopropancarbonsäurehydrochlorid (ACC HCl) über das Alkalisalz der 1-Aminocarbonylcyclopropancarbonsäure

### Beispiel 1:

Man gibt 143 g (1 mol) 1-Aminocarbonylcyclopropancarbonsäuremethylester in 540 g 7,4%ige Natronlauge und erwärmt die Suspension langsam unter Rühren auf 40 °C. Nach 20 min ist das Reaktionsgemisch homogen und man läßt auf Raumtemperatur abkühlen. Diese Lösung wird für die nächste Stufe bereitgehalten (Lösung A). (Das bei der Reaktion freigewordene Methanol kann vor dem Einsatz der Lösung in die nächste Stufe auch abdestilliert werden.)

Die andere für die nächste Stufe notwendige Lösung wird aus 756,6 g (1,25 mol) 12,3%iger Natriumhypochloritlösung und 267 g (2 mol) 30%iger Natronlauge hergestellt (Lösung B).

Lösung A und B werden jeweils bei Raumtemperatur innerhalb von 1 h in ein Mischgefäß gepumpt und anschließend durch einen Rohrreaktor geführt, der auf 80 °C temperiert ist (Verweilzeit: 3,5 min). Der homogene Reaktionsaustrag besitzt einen pH-Wert von 11. Durch Zugabe von 400 ml (4 mol) konzentrierter Salzsäure stellt man den pH-Wert des Gemisches auf 1 ein. Die saure Lösung wird daraufhin zur Trockne eingedampft, wobei die Sumpftemperatur 60 °C nicht überschreiten soll. ACC HCl wird nun mit 4 800 ml Ethanol aus dem Rückstand herausgelöst und durch Filtration vom Natriumchlorid getrennt. Der Ethanolextrakt wird ebenfalls zur Trockne eingedampft, der Rückstand in 200 g heißem Aceton gerührt und anschließend filtriert. Nach der Trocknung des Filterrückstandes erhält man 110 g (80 %) ACC HCl (Reinheit: 98 %).

### Beispiel 2:

a) In eine Lösung von 1 106 g (7 mol) Cyclopropan-1,1-dicarbonsäuredimethylester in 700 ml Methanol wird bei Raumtemperatur unter Rühren Ammoniak (20 l/h) eingeleitet. Nach ca. 10 h beendet man die NH₃-Einleitung.
b) Zu der Suspension gibt man 1,4 1 20%ige Natronlauge und erwärmt das Gemisch auf 40 °C. Im Vakuum wird das Methanol abdestilliert. Man erhält dabei Lösung A.
   Die andere für die nächste Stufe notwendige Lösung wird aus 756,6 g (1,25 mol) 12,3%iger Natriumhypochloritlösung und 267 g (2 mol) 30%iger Natronlauge hergestellt (Lösung B).
c) Lösung A und B werden jeweils bei 20 °C innerhalb von 1 h in ein Mischgefäß gepumpt und anschließend durch einen Rohrreaktor geführt, der auf 80 °C temperiert ist (Verweilzeit: ca. 3,5 min). Der homogene Reaktionsaustrag besitzt einen pH-Wert von 11. Durch Zugabe von 400 ml (4 mol) konzentrierter Salzsäure stellt man den pH-Wert des Gemisches auf 1 ein. Die saure Lösung wird daraufhin zur Trockne eingedampft, wobei die Sumpftemperatur 60 °C nicht überschreiten soll. ACC HCl wird nun mit 4 800 ml Ethanol aus dem Rückstand herausgelöst und durch Filtration vom Natriumchlorid getrennt. Der Ethanolextrakt wird ebenfalls zur Trockne eingedampft, der Rückstand in 200 g heißem Aceton gerührt und anschließend filtriert. Nach der Trocknung des Filterrückstandes erhält man 725 g (76 %) ACC HCl (Reinheit: 98 %).

## Patentansprüche

1. Verfahren zur Herstellung von 1-Aminocyclopropancarbonsäurehydrochlorid der Formel 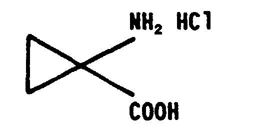 aus einem Cyclopropan-1,1-dicarbonsäurediester,
dadurch gekennzeichnet,
daß man einen Cyclopropan-1,1-dicarbonsäurediester der Formel 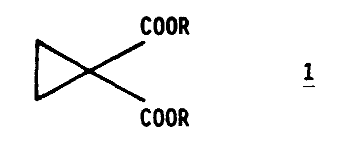 über die Zwischenprodukte 1-Aminocarbonylcyclopropancarbonsäureester der Formel 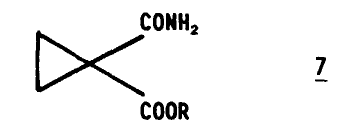 und 1-Aminocarbonylcyclopropancarbonsäuresalz der Formel 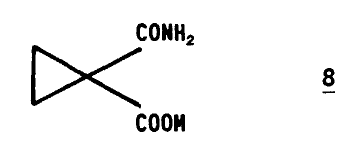 umsetzt, wobei R Alkyl mit 1 bis 8 C-Atomen und M Alkali- oder Erdalkalimetall bedeuten und die Reaktion vom Cyclopropan-1,1-dicarbonsäurediester der Formel **1** zum 1-Aminocarbonylcyclopropancarbonsäureester der Formel **7** in einem Alkohol mit 1 bis 8 C-Atomen mit Ammoniak bei einer Temperatur von 0 bis 100 °C und einem Ammoniakdruck von 1 bis 5 bar durchgeführt wird.

2. Verfahren nach Anspruch 1,
dadurch gekennzeichnet,
daß man die Umsetzung durch Alkalimetall- oder Erdalkalimetallalkoholate katalysiert.

3. Verfahren nach Anspruch 1,
dadurch gekennzeichnet,
daß man den 1-Aminocarbonylcyclopropancarbonsäureester der Formel **7** mit einem Äquivalent einer wäßrigen Alkalihydroxidlösung zum Alkalisalz der 1-Aminocarbonylcyclopropancarbonsäure gemäß Formel **8** verseift.

4. Verfahren nach Anspruch 3,
dadurch gekennzeichnet,
daß die anschließende Umsetzung des Alkalisalzes der 1-Aminocarbonylcyclopropancarbonsäure kontinuierlich oder halbkontinuierlich mit einem Gemisch aus Alkalihypochlorit und einer Alkalihydroxidlösung bei 40 bis 150 °C erfolgt.

5. Verfahren nach Anspruch 4,
dadurch gekennzeichnet,
daß man das Umsetzungsprodukt mit Salzsäure auf pH 1 bis 2 einstellt und aus der wäßrigen Lösung das 1-Aminocyclopropancarbonsäurehydrochlorid in fester Form isoliert.

6. Verfahren nach Anspruch 1 oder 4,
dadurch gekennzeichnet,
daß man die Zwischenstufen **7**, **8** und gegebenenfalls das Produkt gemäß Anspruch 4 nicht isoliert, sondern direkt in die jeweils nächste Stufe einbringt.

## Claims

1. A process for the preparation of 1-aminocyclopropanecarboxylic acid hydrochloride, of the formula 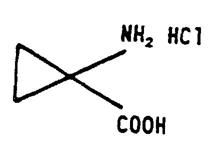 from a cyclopropane-1,1-dicarboxylic acid diester, characterized in that a cyclopropane-1,1-dicarboxylic acid diester of the formula 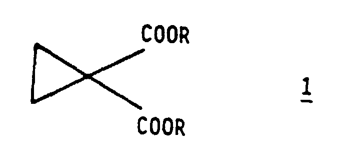 is reacted via the intermediates 1-aminocarbonylcyclopropanecarboxylic acid ester of the formula 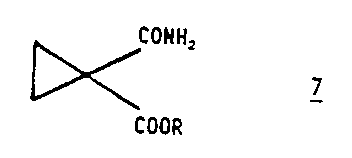 and 1-aminocarbonylcyclopropanecarboxylic acid salt of the formula 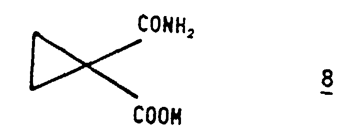 where R is alkyl having 1 to 8 C atoms and M is alkali metal or alkaline earth metal and the reaction of the cyclopropane-1,1-dicarboxylic acid diester of the formula 1 which gives the 1-aminocarbonylcyclopropanecarboxylic acid ester of the formula 7 is carried out with ammonia at a temperature of from 0 to 100°C and an ammonia pressure of 1 to 5 bar in an alcohol having 1 to 8 C atoms.

2. A process according to claim 1, characterized in that the reaction is catalysed by alkali metal alcoholates or alkaline earth metal alcoholates.

3. A process according to claim 1, characterized in that the 1-aminocarbonylcyclopropanecarboxylic acid ester of the formula 7 is hydrolysed with an equivalent of an aqueous alkali metal hydroxide solution to give the alkali metal salt of 1-aminocarbonylcyclopropanecarboxylic acid in accordance with formula 8.

4. A process according to claim 3, characterized in that the subsequent reaction of the alkali metal salt of the 1-aminocarbonylcyclopropanecarboxylic acid proceeds continuously or semicontinuously with a mixture of alkali metal hypochlorite and an alkali metal hydroxide solution at 40 to 150°C.

5. A process according to claim 4, characterized in that the pH of the reaction product is brought to 1 to 2 using hydrochloric acid and the 1-aminocyclopropanecarboxylic acid hydrochloride is isolated in solid form from the aqueous solution.

6. A process according to either of claims 1 and 4, characterized in that the intermediates 7, 8 and, if desired, the product as set forth in claim 4, are not isolated but introduced directly into in each case the next step.

## Revendications

1. Procédé de préparation de chlorhydrate d'acide 1-aminocyclopropanecarboxylique de formule 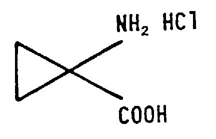 à partir d'un diester d'acide cyclopropane-1,1-dicarboxylique, caractérisé en ce que l'on fait réagir un diester d'acide cyclopropane-1,1-dicarboxylique de formule 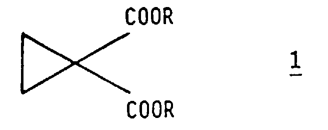 en passant par les produits intermédiaires qui sont l'ester de l'acide 1-(aminocarbonyl)cyclopropanecarboxylique de formule 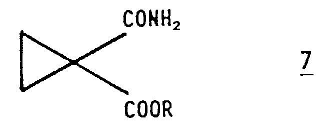 et le sel de l'acide 1-(aminocarbonyl)cyclopropanecarboxylique de formule 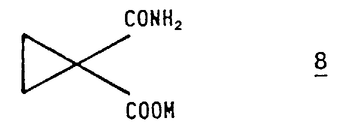 où R représente un résidu alkyle comportant de 1 à 8 atomes de carbone et M représente un métal alcalin ou alcalino-terreux, et en ce que la réaction du diester de l'acide cyclopropane-1,1-dicarboxylique de formule 1 donnant l'ester de l'acide 1-(aminocarbonyl)cyclopropanecarboxylique de formule 7 est réalisée dans un alcool en C₁₋₈ en présence d'ammoniac à une température de 0 à 100°C et à une pression d'ammoniac comprise entre 1 et 5 bars.

2. Procédé conforme à la revendication 1, caractérisé en ce que la réaction est catalysée par des alcoolates de métaux alcalins ou alcalino-terreux.

3. Procédé conforme à la revendication 1, caractérisé en ce que l'on convertit l'ester d'acide 1-(aminocarbonyl)cyclopropanecarboxylique de formule 7 en sel alcalin d'acide 1-(aminocarbonyl)cyclopropanecarboxylique de formule 8 par saponification avec un équivalent d'une solution aqueuse d'hydroxyde de métal alcalin.

4. Procédé conforme à la revendication 3, caractérisé en ce que l'on effectue la réaction subséquente du 1-(aminocarbonyl)cyclopropanecarboxylate de métal alcalin avec un mélange d'hypochlorite de métal alcalin et d'une solution d'hydroxyde de métal alcalin en continu ou en discontinu à une température comprise entre 40 et 150 °C.

5. Procédé conforme à la revendication 4, caractérisé en ce que l'on ajuste par addition d'acide chlorhydrique le pH du produit réactionnel à une valeur comprise entre 1 et 2 et en ce que l'on isole le chlorhydrate d'acide 1-aminocyclopropanecarboxylique sous forme solide à partir de la solution aqueuse.

6. Procédé conforme à la revendication 1 ou 4, caractérisé en ce que l'on omet l'isolement des produits intermédiaires 7 et 8 et éventuellement du produit conforme à la revendication 4, mais en ce que l'on l'utilise directement pour l'étape suivante.
